# EUROPEAN PATENT APPLICATION

(11) **EP 1 878 770 A1**
(43) Date of publication of application: **16.01.2008**
(21) Application number: 06116732.6
(22) Date of filing: 06.07.2006
(51) Int. Cl.: C09B 47/04, C08K 5/3417

(54) **Cyanophthalocyanine derivatives**

(71) Applicant: ChemIP B.V., 6167 RD Geleen (NL)
(72) Inventor: Brokken-Zijp, Josephina, 5298 LT, Liempde (NL)
(74) Representative: Mulder, Cornelis A.M.

(57) **Abstract**

Aminocyanophthalocyanine derivatives of the formula PcM.(CN).(NH₃), in which PcM represents: 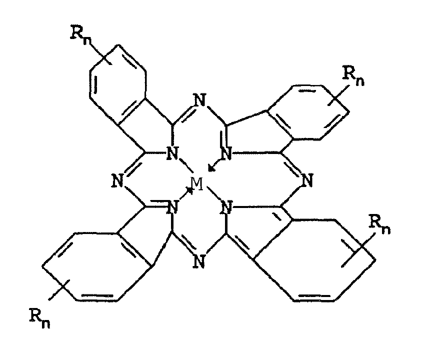 wherein each R independently represents a substituent; each n independently represents an integer from 0 to 4; and M represents Co, Fe, Mn or Cr; also claimed is a process for preparation of aminocyanophthalocyanine cobalt derivatives and the use of the derivatives to obtain conductive polymers.

## Description

The present invention relates to certain cyanophthalocyanine derivatives, to a process for preparing these derivatives and to their use in polymer compositions.

Apart from their use as dyes, nucleating agents or explosion prohibition agent, aquocyanophthalocyanine derivatives of transition metals find use in the preparation of conductive additives for use in a broad range of polymers. In this respect reference is made to e.g. patent application WO-A-93/24562. To this end the aquocyanophthalocyanine particles are added to polymers, and it is believed that these particles form networks in the polymer matrices and thereby infer conductivity to the polymer compositions thus obtained. Polymers in which these aquocyanophthalocyanine derivatives can be used include epoxy resins, polyolefins such as polyethylene, polypropylene, polystyrene which may or may not be expanded, rubbers, such as styrene-butadiene block copolymers and engineering plastics such as polyketones and polyamides.

A process for the preparation of cyanophthalocyanine derivatives has been described by Hanack et al. (see e.g. J. Amer. Chem. Soc. 105 (1983) 828-830). In WO-A-93/24562 a further process for the preparation of aquocyanophthalocyanine derivatives is disclosed. In an example of the latter process a phthalocyaninato cobalt(II) is contacted with an oxidant and an excess of sodium cyanide in a mixture of ethanol and water to yield sodium dicyanophthalocyaninato cobalt (III). By refluxing in water the latter product is subsequently converted to aquocyanophthalocyaninato cabalt(III) which can be used to infer conductivity to a polymer. In this reaction salts are also formed as by-product. If a high-purity product is desired, these byproducts need to be removed by washing.

The aquocyanophthalocyanine derivatives and their networks in polymer compositions known in the art show the disadvantage that sometimes they either decompose or do not mix well in polymers. It has now surpringly been found that certain other cyanophthalocyanine compounds have a higher thermal stability and/or show a better dispersion behavior in thermoplastic polymers resulting in excellent permanent antistatic/static control properties for these phthalocyanine polymer blends made.

Accordingly, the present invention provides aminocyanophthalocyanine derivatives of the formula PcM.(CN).(NH₃), in which PcM represents: 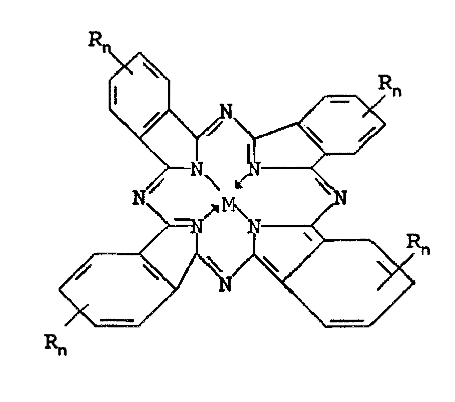 wherein each R independently represents a substituent; each n independently represents an integer from 0 to 4; and M represents Co, Fe, Mn or Cr.

The structure of these compounds can be represented as follows (for M = Co):

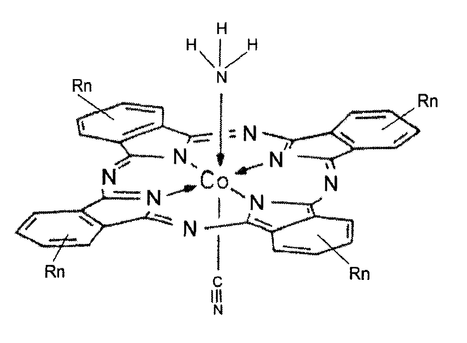

As indicated above, the phthalocyanine moiety Pc may contain substituents. Suitable substituents R include the small substituents disclosed in DE-A-32 45 750. In this reference suitable substituents are described, selected from the group comprising optionally halogenated alkyl or alkoxy groups with 1-10 carbon atoms, naphtacene, trimethylsilyl, a nitro group and a sulphate (SO₃H) group. Other suitable substituents include a cyano, an optionally alkylated amino or halogen moiety. The most suitable halogen is fluorine. The substituents methyl, t-butyl, methoxy, nitro, cyano, amino and/or sulphate are preferred. Because the substituents to a degree may interfere with the conductivity of the eventual phthalocyanine derivative, it is most preferred to employ unsubstituted Pc groups, i.e. when each n is 0.

Because the conductivity effect provoked by cobalt and iron complexes are perfectly adequate and the PcCo and PcFe compounds are relatively easy to obtain in a desired purity, cobalt and iron compounds are preferred.

The present invention further provides a process for the preparation of aminocyanophthalocyanine derivatives of formula PcCo.(CN).(NH₃) in which Pc, R, and n have the meaning as described above, in which process a dicyanophthalocyanine alkali salt of formula [PcCo.(CN)₂]X, in which X represents an alkali metal, is heated in the presence of ammonia or an ammonium salt. These dicyanophthalocyanine starting materials are known from the Hanack article referred to above and from WO-A-93/24562.

The cyanophthalocyanine cobalt derivatives of the present invention preferably are prepared according to a process comprising the steps of
1 providing a dicyanophthalocyanine compound of formula [PcCo.(CN)₂]X in a suitable liquid in the presence of an ammonium source
2 heating the reaction mixture to a temperature between 50 and 200 °C under efficient agitation
3 isolating the produkt from the reaction mixture
4 optionally washing the reaction product

The process according to the invention is preferably carried out under an inert atmosphere, whereby preferably also the presence of oxygen in the liquid is avoided. The liquid preferably is deoxygenated by means known to the skilled man. During the reaction an inert gas, such as nitrogen or argon, may be passed over the reaction mixture. Preferably the liquid contains less then 10 ppm oxygen (O₂), more preferably less then 5 ppm.

Heating of the dicyanophthalocyanine alkali salt is suitably affected in a liquid. That ensures a good heat transfer, avoids the risk of overheating the compounds, and provides a medium for dissolving and/or removing of contaminants. Although the phthalocyanine alkali salts have a limited solubility in water, water is preferably used as liquid. It has the advantage that it dissolves the ammonia or ammonium salt and also the by-products such as alkali metal salts It further ensures a sufficiently high temperature at reflux conditions. However, it is possible to use a reaction medium in which water has been mixed with other water-miscible solvents, such as alcohols, in particular C₁-C₃ alcohols, ketones, such as acetone, acetonitril, tetrahydrofuran etc. However, it is most convenient to use water.

The pH of the mixture in which the reaction is conducted is not critical. Suitably it will be at least 4, more suitably at least 6. Preferred pH values include those from 8 to 12.

The temperature at which the reaction is carried out is suitably above 50 °C. Preferably, the reaction temperature ranges from 70 to 140 °C, with more preference the reaction temperature is at or near the reflux temperature of the reaction mixture, which, when water is being used as liquid at ambient pressure, will be between 95 and 105 °C. The advantage is that at the reflux temperature the reaction rate is sufficiently fast to be economically attractive. Higher temperatures and higher pressures may be also used.

The process preferably is carried out with efficient agitation, that will increase the speed of reaction. Agitation can be carried out by mechanical stirring, boiling the liquid, passing a gas through the liquid and other means of agitation known to the skilled man.

It will be appreciated that, by extensive washing of the dicyanophthalocyanine alkali salt with water and/or alcohol and subsequent drying of the washed product, the dicyanophthalocyanine compound will be converted into the intermediate compound [PcM.(CN).(Y)]X in which Y represent OH⁻ or an alcoholate ion, e.g. ethoxylate, methoxylate or propoxylate. The latter conversion has been described in WO-A-93/24562. Also the intermediate compound can be dissolved in acetone (by for example extraction) and after evaporation of the acetone the intermediate product of which the alcoholate ion and/or OH ion is, at least partly, replaced by the acetone dimer ion, is formed. Although this conversion route is possible it does not appear to entail any particular advantage over the conversion of the dicyanophthalocyanine alkali salt directly. However, it is possible to convert the dicyanophthalocyanine alkali salt [PcM.(CN)₂]X into an intermediate compound, which intermediate compound is subsequently converted into the aminocyanophthalocyanine compound of the present invention by heating in the presence of ammonia or an ammonium salt.

The above processes are carried out in the presence of an ammonium source. Suitable ammonium sources are ammonia or an ammonium salt. In principle any ammonium salt can be used, e.g. ammonium carbonate, ammonium sulphate, ammonium nitrate, but also ammonium salts of organic acids, such as ammonium acetate. It will be appreciated that (aqueous) ammonia will be generated in situ when an ammonium salt, like those described above, is added to an alkaline aqueous liquid. Hence, if the heating step is carried out in the presence of an ammonium salt and a basic compound a reaction mixture emerges that contains aqueous ammonia.

Another way for creating ammonia is the hydrolysis of cyanide ions under conditions of a high pH, like for example a pH between 8 and 14. The cyanide ion may be converted in an aqueous environment into ammonia and a formiate ion. One potential source of cyanide ions can be the presence of cyanide salts in the dicyanophthalocyanine starting material. As indicated above this starting material may be washed. By this washing step cyanide salts may be removed. However, if no washing or insufficient removal of cyanide salts is being done the remaining cyanide salts may be hydrolysed and form ammonia and formiate during the synthesis of the compound of the invention. Moreover, also during the reaction to form the compound of the present invention alkali metal or hydrogen cyanide may be formed. This can also be hydrolysed during the reaction. When the cyanide ions are allowed to stay in the reaction mixture, e.g. if the reaction mixture is not thoroughly stripped or distilled, the amount of ammonia may become such that it reacts with the dicyanophthalocyanine intermediate compound to yield the amino cyanophthalocyanine compound PcM.(CN).(NH₃).

The process of the present invention may also form mixtures of PcM.(CN).(NH₃) and PcM.(CN).(H₂O) when the amount of NH₃ is less then stoechiometric. For example, when no additional ammonia is added to a reaction mixture comprising cyanide ions in a basic environment, the formation of such mixtures may occur. Surprisingly both compounds form mixed crystals when they are formed simultaneously. The invention therefore further provides a mixed crystal comprising PcM.(CN).(NH₃) and PcM.(CN).(H₂O) in which Pc, R, n and M have the meanings as defined above. The concentration of the ammonia used may vary. If a almost complete conversion is desired at least a stoichiometric amount of ammonia or ammonium salt is used. However, if a mixed crystal is desired substoichiometric amounts may be employed. Suitably, the molar ratio of ammonia or ammonium salt to PcM is from 1:0.01 to 1:100. Preferably the ratio is between 1:0.01 and 1:50, more preferably between 1:0.05 and 1:20.

The ratio of PcM.(CN).(NH₃) and PcM.(CN).(H₂O) in these crystals may vary. It has been found that both compounds have a comparable crystal structure, which makes it possible to make mixed crystals of both compounds. The crystals of PcM.(CN).(NH₃) have a unit cell size in which a = 0.73 nm ± 0.03 nm; b = 2.5 ± 0.1 nm; c = 0.72 ± 0.03 nm; β = 103° ± 2 °; α = γ = 90 ± 2°. The crystals have the form of a tile, the two sides having about the same size, the height of the tile being approximately 0.2 times the side of the tile. When hereinafter reference is made to the size of the crystals, the size of the side of the tile is mentioned. For example when the crystal has a size of 200 nm, this means that the two sides of the crystal are approximately 200 nm long, and the height of the crystal is approximately 40 nm. The ratio of both complexes in the mixed crystal may vary broadly, e.g. from 99:1 to 1:99 %m/m.

It has surprisingly been found that the average size of the crystals containing PcM.(CN).(NH₃) and/or PcM.(CN).(H₂O) may be regulated by forming these crystals in the presence of ammonia or ammonium ions in the medium in which these crystals emerge. It has found that the average crystal size becomes bigger when the formation of PcM.(CN).(Z) (with Z being NH₃ or H₂O) is carried out in the presence of ammonia or ammonium ions. Whereas the largest diameter of PcM.(CN).(Z) crystals formed in a medium in which ammonia or ammonium ions are absent, ranges from about 30 to 200 nm, the largest diameter size of such crystals may be up to 800 nm when the formation of crystals is carried out in the presence of NH₃. Preferably the size of these crystals is between 250 and 750 nm.

The reaction time may vary between broad ranges dependent on other reaction conditions as will be appreciated by the person skilled in the art. Suitable reaction times include from 3 to 72 hours. The use of longer reaction times will have an effect on the conversion of starting materials and may also have an effect on the size of the crystals that are being formed of the desired reaction product.

As already indicated earlier the presence of NH₃ may be accomplished by the hydrolysis of cyanide ions in the aqueous medium in which the conversion is usually carried out. It does mean that if small crystals of PcM.(CN).(H₂O) are desired, the presence of ammonia needs to be prevented. It is therefore preferable in such cases to wash the starting material thoroughly to remove any cyanide by-product and to subject the reaction medium to distillation during conversion of PcM(CN)₂Na to PcM.(CN).(H₂O) in order to remove any ammonia formed.

The products of the present invention may be used in polymer compositions to infer conductivity to these compositions. Preferably these polymers or articles made from the polymers have a volume conductivity of at least 1x10 ⁻¹² S/cm.

The present invention therefore also provides polymer compositions comprising a polymer and a cyanophthalocyanine compound of the formula PcM.(CN).(NH₃), in which Pc and R, n and M have the meanings as defined above. It is understood that the present invention also covers polymer compositions comprising a polymer and the cyanophthalocyanine compound of the formula PcM.(CN).(NH₃) in which the cyanophthalocyanine compound is present in a mixed crystal of PcM.(CN).(NH₃) and PcM.(CN).(H₂O).

The polymer in which the present compounds may be used can be selected from a wide range of elastomeric, thermosetting and thermoplastic polymers. For any polymer composition for which conductivity, e.g. for packaging purposes, is required or desired it is possible to include a minor amount of the present compound in the composition. Because only minor amounts are needed to achieve the desired conductivity the processing properties of the polymer compositions employed are not greatly affected by the addition of the present compound. Examples of suitable polymers include elastomers such as block copolymers of polystyrene and polydiene e.g. polybutadiene or polyisoprene, which polydiene blocks may or may not have been (selectively, say from 80 to 99.5%) hydrogenated, or to which functionalised groups like carboxylic groups may have been attached, and neoprene. Other suitable polymers include polyolefins like polyethylene, polypropylene, poly(iso)butylene, polystyrene, and (random)copolymers of such olefins. Polyvinyl alcohol, polyvinyl chloride, polyvinyl acetates, polyacrylates and polymethacrylates in which the ester groups may be alkyl groups with 1 to 6 carbon atoms, may also be used. Other suitable polymers are polyketones, e.g. copolymers of carbon monoxide and ethylene and/or propylene, polyamides, nylons, polycarbonates, polyesters, such as PET (polyethylene terephthalate), epoxy resins, polyphenylethers, polyurethanes and polysulphones. Natural polymers such as cellulose and natural rubber, may also be used. Preferred polymer compositions include epoxy resins, poly(meth)acrylates,; polyolefins, such as polyethylene and polypropylene; polystyrene; elastomeric styrene/butadiene or isoprene block copolymers optionally hydrogenated and/or functionalised; polyesters; polyamides; PVC and polyketones obtained by the polymerisation of carbon monoxide and an olefin such as ethylene and/or propylene.

The novel compositions of this invention are intended for further processing by any of the techniques known per se. The latter include technology such as fiber spinning, compression moulding, injection moulding, co-extrusion, laminating, blow-moulding, solid phase pressure forming, vacuum forming, deep drawing, powder coating, solution casting, casting, prepegging and the like. The envisaged final articles of manufacture can be selected from fibers, monofilaments, yarns, coatings, pipes, tubes, tyres, films, sheetings, non-woven fabrics, various mouldings, castings and laminates. Antistatic or static control films made from thermoplastic polymers or antistatic or static control floorings or coatings produced from thermosetting matrices are also very attractive.

The amount of the present compound in the polymer compositions of the invention may be selected dependent on the desired use of the polymer composition. In general a sufficient level of conductivity, e.g. for permanent anti-static control of floor coverings and permanent anti-static control of packaging, can be obtained with relatively small amounts of the cyanophthalocyanine compounds of the present invention. Amounts may range from 0.05 %wt upwards. The amounts of the present compound in the polymer compositions to be used as floor coverings are therefore suitably from 0.05 to 20 %wt, based on the weight of the polymer and the cyanophthalocyanine compound, preferably from 0.1 to 5 %wt. These amounts compare favourably to the amount of carbon black in e.g. floor coverings. There a sufficient anti-static effect can be obtained with about 20 to 30 %wt of carbon black, based on the weight of the polymer and the carbon black.

If the compounds are to be used as nucleating agent the amount may suitably be from 0.05*10⁻³ to 1.0 %wt. For use as dyes, the amounts required may be higher, e.g., from 0.05*10⁻³ to 20%wt. When the compounds are used to improve the mechanical properties of the polymer matrix the amounts required may be higher, e.g., from 0.5 to 40%wt.

It will be evident that mechanical properties of the polymers are less affected when smaller amounts of cyanophthalocyanine compounds are added. In practice the compounds will often be handled as master batches, i.e., concentrated mixtures of the cyanophthalocyanine compound according to the invention with a polymer. The concentration of the compound in masterbatches may be from 3 to 90%wt, based on the total of compound and polymer. The use of larger particles, e.g. when the products of the present invention are being used instead of aquocyanophthalocyanine compounds, has an advantage in relation to the preparation of a master batch and to the preparation of an attractive powder blend. It has unexpectedly be found, that preparation of masterbatches and thermoplastic polymer compositions can be performed more easily with the compounds of the present invention. The larger sized particles of the compounds of the present invention can more easily be sheared, whereby masterbatches can be made with polymer matrices that cannot withstand large shear forces. Also the stability of the compounds of the present invention is larger, whereby mixing can be performed at higher temperatures without deterioration of the compounds of the invention.

The polymer compositions of the present invention may further contain other common additives that may be useful for the purpose for which they are used. The polymer compositions may be employed for making a broad range of products including injection-moulded or blow-moulded articles, coatings, fibres and films. It has been found that stretching of the polymer compositions into films may leave the networks of the compound of the present invention unaffected, and therefore these films retain their permanent anti-static control properties.

The compounds of the present invention may also be used in a variety of other applications like for example a powder additive for prohibition of explosions; Antistatic sprays for making permanent antistatic paper, prevent shocks to persons (spaying hands) or to prevent dust build-up on surfaces; as pigments for all kind of applications like for example inks. The particles of the compounds of the present invention may also act as reinforcing fillers in polymers and may also be applied with different particle sizes (like for example bimodal particle size distributions).

The invention will be further illustrated by means of the following examples.

### EXAMPLE 1

In a closed three-litre reactor 157.14 g of PcCo(CN)₂.Na, 24.88 gr of a 25% NH₃ solution in water and 934.2 g demineralised water was heated at 90°C under nitrogen. After 30 hours the reaction was stopped, the solid product formed was filtered, washed twice with demineralised water and dried for three days at 80°C under vacuum. The product was PcCo.(CN).(NH₃) in a yield above 90%. The gaseous products found at temperatures above 150°C were measured using Mass Spectrometry (MS) and Fourier Transformation-Infra-Red (FTIR) techniques: the products included NH₃, HCN and (CN)₂, but no H₂O was found. From these data it was concluded that the product only contained the PcCo.(CN).(NH₃) product. The product shows a high thermal stability: It was found that the product lost its NH₃ ligand at about 250 °C.

Elemental analysis of the product yielded C 64.25% (theory 64.49); H 22.73% (theory 22.80); N 3.06% (theory 3.12); Co 9.52% (theory 9.59).

X-ray diffraction analysis showed a unit cell of comprising a = 0.739 ± 0.001 nm, b = 2.462 ± 0.002 nm; c = 0.733 ± 0.001 nm; α = γ = 90°; β = 104.6 ± 0.1 °. The average particle size was 340 ± 40 nm. The powder conductivity measured between two steel electrodes under 5 bar pressure amounted to 1.4*10⁻³ S/cm.

### EXAMPLE 2

191 gr PcCo(CN)₂.Na precursor in about 1 litre of demineralised water was heated in a closed 2 litre reactor for 24 hours at 95°C. The solid product produced was filtered off, washed twice with demineralised water and dried for three days under vacuum at 80°C. The yield was over 90%wt.

A sample of the solid product obtained was heated in nitrogen and in the absence of water between 40 and 300°C with a heating rate of 10°C/min while the mass of the sample was recorded. At temperatures above 150°C the product starts loosing its ligands, but the phthalocyanine ring itself does not decompose. The gaseous products found at temperatures above 150°C were measured using Mass Spectrometry (MS) and Fourier Transformation-Infra-Red (FTIR) techniques. The products were found to be NH₃, H₂O HCN and (CN)₂. The latter products were formed from the cyano ligand. From these data it was concluded that the molar ratio of NH₃ to H₂O in the PcCo.(CN).(NH₃/H₂O) containing sample was about 30:70. X-Ray diffraction analysis of the product indicated a unit cell with a = 0.735 ± 0.003 nm; b = 2.471 ± 0.002 nm; c = 0.724 ± 0.001 nm; α = γ = 90°; β = 104.1 ± 0.1°. The average particle size was 270 ± 30 nm.

The powder conductivity was found to be 2.0 10⁻³ S/cm under the circumstances indicated in Example 1.

### COMPARATIVE EXPERIMENT 1

The procedure of Example 2 was repeated with the exception that the reaction mixture was heated to 100°C under distillation conditions so that any ammonia that was formed due to hydrolysis of cyanide, would be removed. The product was filtered, washed and dried. The product was the aquocyanophthalocyaninatocobalt compound PcCo.(CN).(H₂O), as described in WO-A-93/24562. A sample of the product was heated from 40 to 300°C in order to establish when the ligands would be released. With the aid of MS and FTIR H₂O and CN could be detected. However, no NH₃ was found.

X-ray diffraction analysis indicated a unit cell with a = 0.728 nm, b = 2.475 nm and c = 0.715 nm; α = γ = 90°; β = 103°. The conductivity of the powder was 1.5*10⁻³ S/cm under the conditions of Example 1.

The temperature at which the compounds loose their ligands is a measure of the stability of the products. The product of Comparative Experiment 1 lost its H₂O ligand at about 220 °C. Hence, the compound of the present invention is more stable than the aquocyanophthalocyanine compound of WO-A-93/24562.

### EXAMPLE 3

A masterbatch containing 30%w of PcCo.(CN).(NH₃/H₂O) in which the ratio NH3:H₂O was about 30:70, in linear low-density polyethylene (LLDPE) was mixed with nibs of commercial LLDPE in a single screw extruder. Films were made by extrusion film blowing at 220°C. The die gap was 1 mm. A blow ratio of three was applied. Films were made with different amounts of cyanophthalocyanine derivatives ("PcCo der") and with different film thicknesses. The conductivity of the films was determined. The results are shown in the following Table 2.

**Table 2**

| Sample | PcCo der (w%) | Film thickness (mm) | Conductivity (S/cm) |
|---|---|---|---|
| 1 | 0.0 | 0.10 | < 1.0*10⁻¹⁵ |
| 2 | 0.5 | 0.10 | 5.0*10⁻¹¹ |
| 3 | 0.5 | 0.15 | 4.2*10⁻¹¹ |
| 4 | 0.5 | 0.05 | 4.5*10⁻¹¹ |
| 5 | 1.0 | 0.10 | 5.0*10⁻¹¹ |

From these results it appears that the conductivity of very thin films and at low concentrations of the additive remains in the same order of magnitude, indicating that the conductive networks of derivative remain intact.

### Example 4

A masterbatch was made (PP masterbatch 1) from 10 w% PcCo.(CN).(NH₃/H₂O) in which the ratio NH3:H₂O was about 35:65 powder (average primary particle size 450 x 450 x100 nm), 15 w% PP-wax and 75 w% of a standard PP under high shear conditions. Before adding the PcCo.(CN).(NH₃/H₂O) powder to the PP masterbatch medium the powder was dried for 3 days at 80 °C under vacuum. Of this mixture standard nibs were made using in the last step a single screw extruder. The distribution of the PcCo.(CN).(NH₃/H₂O) particles in these masterbatch nibs were analyzed using Confocal Scanning Laser Microscopy (CSLM) and Optical Microscopy (OM; slice thickness about 7 µm). and the volume conductivity was measured using the four point method (ASTM D 991). With CSLM fractal particle networks of the PcCo.(CN).(NH₃/H₂O) particles were observed and the volume conductivity of this masterbatch was 2X10⁻¹¹ S/cm.

### Example 5

PP masterbatch 1 was mixed with PP nibs (Shell grade KY 6100) on a roller mill for 6 minutes (temperature 170,5 °C). The product was moulded between glass plates and the blend and the distribution of the PcCo.(CN).(NH₃/H₂O) in which the ratio NH3:H₂O was about 35:65 particles were analyzed using CSLM and Optical Microscopy The concentrations used of MB was 5.0, 10.0, and 20.0 % ,which corresponds to respectively 0.5, 1.0, 2.0 w% PcCo.(CN).(NH₃/H₂O) in the PP blend. All these blends were conductive (volume conductivity 1 - 3x10⁻¹¹ S/cm). Fractal PcCo.(CN).(NH₃/H₂O) particle networks were observed with CSLM and OM (magnification 500 - 1000X). The PcCo.(CN).(NH₃/H₂O) particle distribution was very similar to the one observed in PP masterbatch 1 itself.

### Comparative experiment 2

A masterbatch was made by mixing under high shear conditions 10 w% of PcCo.(CN).(H₂O) powder (average primary particle size 100 x 100x 20 nm) with 15 w% PP wax and 75 w% PP nibs (PP masterbatch 2). Of this mixture standard nibs were made using in the last step a single screw extruder. For making this masterbatch the same composition was used and the same processing route was followed as for PP masterbatch 1 mentioned under example 4. The distribution of the PcCo.(CN).(H₂O) particles in the nibs of this masterbatch 2 and the volume conductivity of these masterbatches were determined as described for PP masterbatch 1. No particle networks were found in these masterbatches and no conductivity could be measured (volume conductivity < 10⁻¹⁵ S/cm on moulds made between glass plates). In the OM pictures a large number of PcCo.(CN).(H₂O) agglomerates were observed of sizes well above 1 µm. Before adding the PcCo.(CN).(H₂O) powder to the PP masterbatch medium the powder was dried for 3 days at 80 °C under vacuum.

### Comparative experiment 3

PP masterbatch 2 was mixed with PP nibs (Shell grade KY 6100) on a roller mill for 6 minutes (temperature 170,5 °C). The blend was moulded between glass plates and the distribution of the PcCo.(CN).(H₂O) particles were analyzed using CSLM and Optical Microscopy The concentrations used of MB was 1.0, 2.0, 5.0, 10.0, and 20.0 %, which corresponds to respectively 0.1, 0.2, 0.5, 1.0, 2.0 w% PcCo.(CN).(H₂O) in the PP blend. None of these blends were conductive (volume conductivity < 10⁻¹⁵ S/cm). Only agglomerates of PcCo.(CN).(H₂O) particles were observed with CSLM and OM. The agglomerates distribution was very similar to the one observed in PP masterbatch 2 itself.

### Example 6

A LLDPE masterbatch (PE masterbatch 1) was made by mixing under high sheer conditions 30 w% PcCo.(CN).(NH₃/H₂O) in which the ratio NH3:H₂O was about 35:65 powder (average primary particle size 450x450x100 nm), 40 w% PE wax 520 and 30 w% of the same LLDPE (Melt Flow Index 20-25 dg/min) (PE masterbatch 1). Of this mixture standard nibs were made using in the last step a single screw extruder. The distribution of the PcCo.(CN).(NH₃/H₂O) particles in these masterbatch nibs were analyzed using Confocal Scanning Laser Microscopy (CSLM), Optical Microscopy (OM; slice thickness about 7 µm). and the volume conductivity was measured using the four point method (ASTM D 991). PcCo.(CN).(NH₃/H₂O) fractal particle networks were found in these masterbatches and the conductivity could be measured (volume conductivity = 2x 10⁻¹⁰ S/cm measured on moulds made between glass plates). Before adding the PcCo.(CN).(NH₃/H₂O) powder to the LLDPE masterbatch medium the powder was dried for 3 days at 80 °C under vacuum.

### Example 7

PE masterbatch 1 was mixed with LLDPE nibs (LLDPE CIPEN LL18EFA) on a roller mill for 5 minutes (temperature 150- 155 °C). The product was moulded between glass plates and the distribution of the semi-conductive phthalocyanine particles were analyzed using CSLM and Optical Microscopy. The concentration used of MB-1 was 3.35 wt%, which corresponds to 1.0 wt% semi-conductive phthalocyanine particles. The blend was conductive (volume conductivity 0.5 - 5x10⁻¹¹ S/cm). Fractal particle networks were observed with CSLM and OM (magnification 500 - 1000X). The particle distribution was very similar to the one observed in the corresponding PE masterbatch itself.

## Claims

1. Aminocyanophthalocyanine derivatives of the formula PcM.(CN).(NH₃), in which PcM represents: 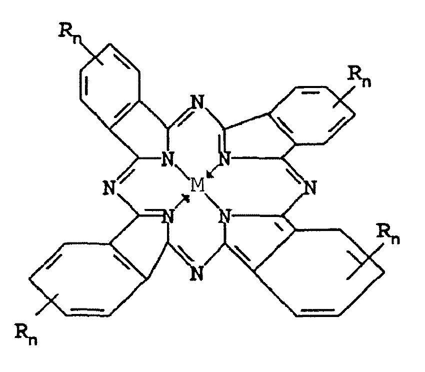 wherein each R independently represents a substituent; each n independently represents an integer from 0 to 4; and M represents Co, Fe, Mn or Cr.

2. The derivatives according to claim 1, wherein the substituents R are selected from the group optionally halogenated alkyl or alkoxy groups with 1-10 carbon atoms, naphthacene, trimethylsilyl, a nitro group and a sulphate (SO₃H) group.

3. The derivatives according to claim 1, wherein n is 0.

4. The derivatives according to anyone of claims 1-3, wherein M = cobalt.

5. The derivatives according to anyone of claims 1-4, wherein mixtures of PcM.(CN).(NH₃) and PcM.(CN).(H₂O) are present.

6. The derivatives according to anyone of claims 1-4, wherein the derivatives are present as crystals having a size between 250 and 750 nm.

7. Process for making aminocyanophthalocyanine cobalt derivatives comprising the steps of
• providing a dicyanophthalocyanine compound of formula [PcCo.(CN)₂]X in a suitable liquid in the presence of an ammonium source
• heating the reaction mixture to a temperature between 50 and 200 °C under efficient agitation isolating the produkt from the reaction mixture
• optionally washing the reaction product.

8. The process according to claim 7, wherein the process is carried out under an inert atmosphere.

9. The process according to claim 7 or 8, wherein the liquid contains less then 10 ppm oxygen (O₂).

10. The process according to anyone of claims 7-9, wherein the liquid comprises water.

11. The process according to anyone of claims 7-10, wherein the liquid has a pH value between 8 and 12.

12. The process according to anyone of claims 7-11, wherein the reaction temperature ranges from 70 to 140 °C.

13. The process according to anyone of claims 7-12, wherein the ammonium source is ammonia or an ammonium salt.

14. Polymer compositions comprising a polymer and a derivative as claimed in anyone of claims 1-6 or prepared by the process according to claims 7-13.

15. Use of cyanophthalocyanine derivatives as described in anyone of claims 1-6 as additives in polymers.

16. Use according to claim 15, for obtaining conductive polymers having a volume conductivity of at least 1x10⁻¹² S/cm.

17. Use of derivatives as claimed in anyone of claims 1-6 as reinforcing filler.

18. Use of derivatives as claimed in anyone of claims 1-6 as uncleating agent.

19. Permanent antistatic article comprising a polymer and a derivative as claimed in anyone of claims 1-6.

20. The article according to claim 19, having a volume conductivity of at least 1x10⁻¹² S/cm.
